# EUROPÄISCHE PATENTSCHRIFT

(11) **EP 3 354 331 B1**
(45) Veröffentlichungstag und Bekanntmachung des Hinweises auf die Patenterteilung: **01.03.2023**
(21) Anmeldenummer: 18152797.9
(22) Anmeldetag: 22.01.2018
(51) Int. Cl.: B01D 61/28, B01D 61/30, A61M 1/16, A61M 1/34

(54) **DIALYSATOR MIT VERBESSERTER INTERNER FILTRATION UND VERFAHREN ZU DESSEN HERSTELLUNG**
DIALYSER WITH IMPROVED INTERNAL FILTRATION AND METHOD FOR PRODUCING THE SAME
DIALYSEUR À FILTRATION INTERNE AMÉLIORÉE ET SON PROCÉDÉ DE FABRICATION

(30) Priorität: 24.01.2017 DE 102017101307
(43) Veröffentlichungstag der Anmeldung: 01.08.2018
(73) Patentinhaber: B. Braun Avitum AG, 34212 Melsungen (DE)
(72) Erfinder: Mandry, Dr. Peter, 01326 Dresden (DE)
(74) Vertreter: Winter, Brandl - Partnerschaft mbB

(56) Entgegenhaltungen:
- DE-T1- 19 782 098
- DE-T2- 69 515 766

## Beschreibung

### Hintergrund der Erfindung

Ziel einer Dialysetherapie ist es, neben der Entgiftung des Blutes, überschüssiges Wasser, welches sich aufgrund eines der Dialyse zugrundeliegenden Nierenversagens im Körper ansammelt, aus diesem zu entfernen. Dies geschieht durch eine sogenannte Ultrafiltration, bei der Flüssigkeit über einen Dialysator aus dem Blut entfernt wird.

Herkömmliche Dialysatoren weisen üblicher Weise ein röhrenförmiges Dialysatorgehäuse mit einer Längsausdehnung auf, wobei der Innenraum des Dialysators einen Querschnitt aufweist, der sich typischer Weise über die gesamte Längsausdehnung nicht oder nur unwesentlich verändert. Im Innenraum sind parallel zueinander angeordnet Kapillarmembranen (Hohlfasermembranen) vorgesehen. Die Kapillarmembranen bilden zusammen einen Abschnitt eines extrakorporalen Blutkreislaufes aus, während der Außenraum der Kapillaren und der Innenraum des Dialysatorgehäuses einen Abschnitt eines Kreislaufs der Dialysierflüssigkeit (Dialysat) ausbilden. Die beiden Kreisläufe sind gegenläufig und über die semipermeablen Membranen der Kapillaren voneinander getrennt. Durch diese semipermeablen Membranen findet sowohl ein Wasserals auch ein Stoffaustausch statt. Insbesondere werden dem Blut des Patienten Wasser und Schadstoffe entzogen. Im Durchmesser oder im Molekulargewicht größer werdende Retentionsprodukte werden bei Dialysatoren durch diffusive Prozesse über die Membranen in schlechterer Weise entzogen als kleinere Schadstoffe.

Als verschiedene Dialysetechniken werden u.a. Hämodialyse, Hämodiafiltration und High-Flux-Dialyse eingesetzt.

Bei der Hämodialyse wird nach dem Prinzip des Konzentrationsausgleichs kleinmolekularer Substanzen zweier Flüssigkeiten verfahren, die durch eine semipermeable Membran getrennt sind (Osmose). Von der Filtermembran getrennt befindet sich auf der einen Seite das Blut mit Elektrolyten wie Kalium und Phosphat sowie harnpflichtigen Substanzen (z.B. Harnstoff, Harnsäure). Auf der anderen Seite der Membran befindet sich eine keimarme, aufbereitete Lösung (Dialysierflüssigkeit), deren Wasser bei Online-Zubereitung durch Umkehrosmose aufbereitet wurde und die keine Abfallprodukte enthält und einen an den jeweiligen Bedürfnissen des Patienten orientierten Anteil an Elektrolyten aufweist. Die semipermeable Filtermembran (Dialysemembran) zwischen Blut und Dialysierflüssigkeit besitzt Poren, die kleine Moleküle wie Wasser, Elektrolyte und harnpflichtige Substanzen durchlassen, aber große Moleküle wie Eiweiße und Blutzellen zurückhalten.

Bei der Hämodiafiltration wird die Hämodialyse und eine Hämofiltration in Kombination angewendet. Dieses Verfahren kommt insbesondere bei chronischer Niereninsuffizienz zur Anwendung und ermöglicht sowohl die Entfernung von nieder- als auch von mittelmolekularen Substanzen bei kontrolliertem Ersatz des Ultrafiltrats durch physiologische Elektrolytlösung (Diluat). Die Ersatzlösung wird entweder dem Blut vor oder nach dem Dialysator zugegeben und im Dialysator wieder entfernt (Ultrafiltration). Dadurch kann ein höherer transmembraner Fluss erzeugt werden, der zur effektiveren Entfernung der Giftstoffe führt.

Schließlich wird unter der High-Flux-Dialyse eine Hämodialyse mit hohem Ultrafiltrationskoeffizienten (K_{UF} > 10) verstanden, der die stündliche Ultrafiltration (Uf) in ml angibt, die pro mmHg Transmembrandruck (TMP) erzielt wird.

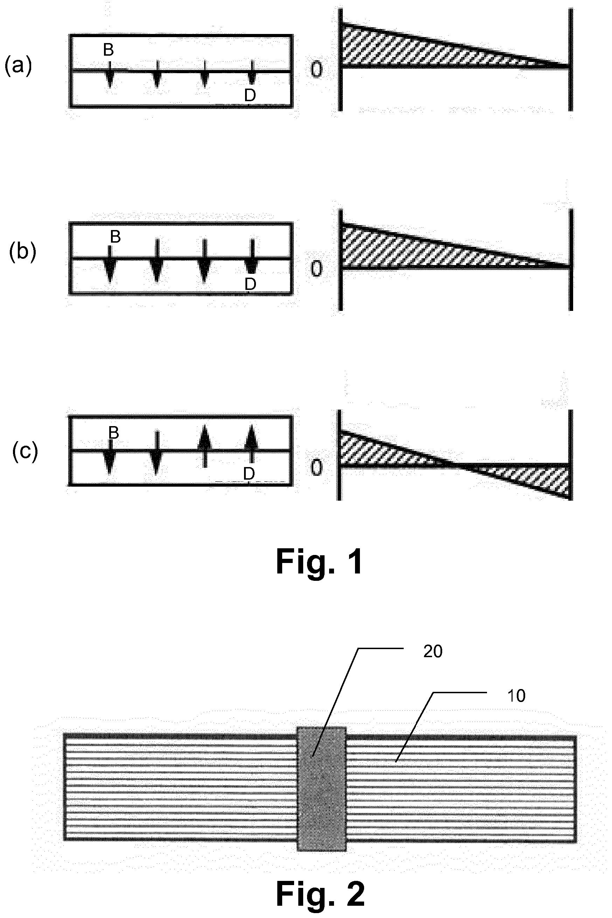

Fig. 1 zeigt schematische Darstellungen der Funktionsweisen der drei vorgenannten Dialysetechniken anhand einer durch Pfeile und deren Größe angedeuteten Diffusionsrichtung und -stärke (linke Diagramme) sowie eines entsprechenden Druckprofils (rechte Diagramme) längs des rohrförmigen Dialysatorgehäuses, nämlich (a) für die Hämodialyse, (b) für die Hämodiafiltration und (c) für die High-Flux-Dialyse.

Wie anhand der Diagramme in Fig. 1(a) erkennbar ist findet bei der normalen Hämodialyse aufgrund der geringen Permeabilität der Membranen trotz positivem TMP-Gradienten eine geringe Ultrafiltration zwischen Blut (B) und Dialysierflüssigkeit (D) statt (geringer Ultrafiltrationskoeffizient). Die in Fig. 1(b) gezeigten Diagramme deuten darauf hin, dass bei der Hämodiafiltration aufgrund des Einsatzes von Membranen mit hoher Permeabilität bei ähnlichem TMP-Gradienten eine deutliche höhere Ultrafiltrationsrate erzielt wird (hoher Ultrafiltrationskoeffizient). Schließlich zeigen die Diagramme in Fig. 1(c), dass sich bei der High-Flux-Dialyse aufgrund einer volumetrischen Steuerung hoher Ultrafiltrationsraten durch das Dialysegerät eine Druckgradientenumkehr entlang des Dialysators ergibt und ein typisches Profil der Filtration/Rückfiltration im Dialysator-Filter erreicht wird. Aufgrund dieses Druckprofils wird die Konvektion im proximalen Bereich des Dialysators (linke Seite des Diagramms) beibehalten (konvektiver Stoff transport), während im distalen Bereich (rechte Seite des Diagramms) durch die mit der Druckgradientenumkehr verbundene Änderung der Diffusionsrichtung eine Rückfiltration (engl.: Backfiltration) stattfindet, mittels der die Ultrafiltration signifikant gesteigert werden kann. Durch die High-Flux-Dialyse kann somit eine konvektive Komponente erreicht werden, so dass eine interne Filtration zur Entfernung von Mittelmolekülen ohne Re-Infusion einer Ersatzlösung möglich ist.

Die Entfernung von sogenannten Mittelmolekülen (Proteine/Proteinfragmente) ist als ein bestimmender Faktor für Unterschiede in der Überlebensrate bei Patienten zu sehen, die entweder mit sogenannten Low-flux-Dialysatoren (K_{UF}<10) zur überwiegend diffusiven Entfernung von kleinmolekularen Teilchen (Molekülen) bei kaum konvektivem Stofftransport von Proteinen, oder mit sogenannten High-flux-Dialysatoren (K_{UF}>10) mit diffusivem und konvektivem Stofftransport zur Entfernung großmolekularer Teilchen (Proteine bis 70 kDa)) behandelt werden. Ein hoher konvektiver Transport von mehreren Litern wird während einer Dialysebehandlung dadurch erreicht, dass eine hohe Ultrafiltrationsrate gewählt und die entfernte Flüssigkeitsmenge des Bluts mittels einer Substitutionsflüssigkeit wieder ersetzt wird. Diese wird entweder aus Infusionslösung oder direkt aus dem Dialysierflüssigkeit gewonnen. Beides ist jedoch mit erhöhten Kosten und apparativem Aufwand verbunden.

### Stand der Technik

Aus der DE 102015100070 A1 ist ein Dialysatorgehäuse bekannt, welches an der Innenseite im Dialysatraum eine ringförmige Verengung besitzt. Durch diese Verengung kommt es zu einem erhöhten Druckabfall der Dialysierflüssigkeit und dadurch zu einer erhöhten Backfiltration.

Ferner ist in Kidney International, Band 54, Ausgabe 3, September 1998 auf den Seiten 979-985 ein Dialysator mit verbesserter konvektiver Leistung durch Einfügen eines O-Rings um ein Membranbündel gezeigt.

Beide vorgenannten Lösungen zur Erhöhung des konvektiven Transportes im Dialysator haben den Nachteil, dass der Herstellungsaufwand sehr hoch ist, und damit die Herstellungskosten erheblich steigen. So erlaubt bspw. die kostengünstige Herstellungsweise des Gehäuses im Spritzguss keinen Hinterschnitt im Innenraum und die dünne Wandstärke keine Einengung des Gehäuses durch Wärme und Kraft von außen. Eine Erhöhung der Wandstärke würde zu einem erhöhten Materialbedarf und längerer Taktzeit führen.

Auch gestaltet sich die Einbringung des O-Ringes in den Innenraum des Dialysators aufgrund der Platzverhältnisse als sehr schwierig. Das Faserbündel (Kapillare) wird mit einer Folie eng ummantelt und in den Dialysator geschoben oder gezogen. Die Folie wird danach herausgezogen. Um eine hohe Clearance (d.h. eine gute Reinigungswirkung) des Dialysators zu erreichen, wird eine möglichst hohe Packungsdichte im Dialysator angestrebt (also eine maximale Anzahl von Fasern im Querschnitt des Dialysators). Dadurch bleibt kein Platz für einen zusätzlichen Ring, der eingeführt werden könnte, zumal das beschädigungsfreie Einführen des Bündels bereits bei herkömmlichen Dialysatoren sehr anspruchsvoll ist.

Die DE 695 125 766 T2 offenbart einen Dialysator mit einem Verengungsabschnitt dessen Füllstoff eingebracht wird, entweder durch Einfüllen eines Harzes oder durch Umwickeln der Hohlfasermembranen mit einem Bündel quellbarer Fasern.

Die DE 197 82 098 T1 offenbart einen Dialysator mit einem Verengungsabschnitt, bei dem der Füllstoff als ein Wasserabsorptionsgel bereitgestellt wird.

### Kurzbeschreibung der Erfindung

Der vorliegenden Erfindung liegt u.a. die Aufgabe zugrunde, den Druckgradienten eines Dialysators bei geringem zusätzlichem Herstellungsaufwand so zu beeinflussen, dass die interne Filtration die Größenordnung einer Hämodiafiltration mit Re-Infusion erreicht.

Diese Aufgabe wird gelöst durch einen Dialysator nach Patentanspruch 1 und ein Herstellungsverfahren nach Patentanspruch 9.

Dementsprechend wird auf einen Teil der Umwickelungsfolie ein Füllstoff aufgebracht, der volumenvergrößernde Eigenschaften aufweist und erst nach dem Einbringen in das Gehäuse expandiert. Dadurch wird das Einführen des Faserbündels in den engen Bereich zwischen Faserbündel und Dialysatorgehäuse erleichtert. Der Füllstoff ist von einer wasserdurchlässigen Folie umgeben, als gelartige Paste ausgebildet oder als in das Dialysatorgehäuse einspritzbares Polymer ausgebildet. Die Entfaltung des Volumens (Aktivierung der Volumenexpansion) kann dann in Abhängigkeit des Füllstoffs durch verschiedene Mechanismen ausgelöst werden. Der expandierte Füllstoff wirkt als Flusswiderstand und erhöht die Druckunterschiede zwischen Dialysierflüssigkeit und Blut.

Somit wird erfindungsgemäß eine einfache Herstellung von Dialysatoren zur Blutreinigung mit gegenüber herkömmlichen Dialysatoren deutlich verbesserter interner Filtration erreicht. Dies führt zu einer besseren Clearance von Mittelmolekülen und damit einer verbesserten Dialysewirkung.

Die vorgeschlagene Lösung kann auch dazu genutzt werden, die Packungsdichte der Fasern über die ganze Länge des Dialysators zu erhöhen, ohne dass dabei beim Einführen der Fasern der Durchmesser des Bündels vergrößert werden muss. Dabei wird nahezu die gesamte Folie bzw. Bündel mit dem Polymer beschichtet.

Spezifische vorteilhafte Ausführungsformen der vorliegenden Erfindung sind in den Unteransprüchen angegeben.

### Figurenbeschreibung

Im Folgenden werden bevorzugte Ausführungsbeispiele der vorliegenden Erfindung unter Bezugnahme auf die beiliegenden Zeichnungsfiguren näher beschrieben. Es zeigen:
Fig. 1 allgemeine Informationen zu bekannten Dialysemethoden,
Fig. 2 ein Faserbündel gemäß einem ersten bevorzugten Ausführungsbeispiel der Erfindung,
Fig. 3 einen schematischen Längsschnitt eines erfindungsgemäßen Dialysators gemäß einem ersten bevorzugten Ausführungsbeispiel der Erfindung in Montierlage,
Fig. 4 einen schematischen Längsschnitt des erfindungsgemäßen Dialysators gemäß dem ersten bevorzugten Ausführungsbeispiel der Erfindung, in fertig montierter Lage,
Fig. 5 einen schematischen Längsschnitt eines erfindungsgemäßen Dialysators gemäß einem zweiten bevorzugten Ausführungsbeispiel der Erfindung in fertig montierter Lage,
Fig. 6 einen schematischen Längsschnitt eines erfindungsgemäßen Dialysators während der Montage und
Fig. 7 einen schematischen Längsschnitt eines erfindungsgemäßen Dialysators gemäß einem dritten bevorzugten Ausführungsbeispiel der Erfindung in fertig montierter Lage.

Im Folgenden werden bevorzugte Ausführungsbeispiele der vorliegenden Erfindung am Beispiel eines High-Flux-Dialysators mit volumenvergrößerndem Füllstoff beschrieben.

In einem ersten Ausführungsbeispiel wird ein in Gegenwart von Wasser quellbarer Füllstoff verwendet, vorzugsweise ein wasserquellbares Polymer. Wasserquellbare Polymere sind beispielsweise aus der DE-A-19748631 bekannt. Besonders bevorzugt sind wasserquellbare Polymere in Form von Homo- oder Copolymeren auf der Grundlage von (Meth)acrylsäure, (Meth)acrylamiden und/oder (Meth)acrylaten, wobei in dem Copolymer beliebige mit den oben angesprochenen Monomeren copolymerisierbare Monomere eingesetzt werden können, welche die Quellfähigkeit des Copolymers nicht beeinträchtigen. Bevorzugte Comonomere sind Acrylnitril, Acrylate, Acrylamide, Allylverbindungen, Vinylacetat, Hy-droxyethylcellulose, Hydroxypropylcellulose, Carboxymethylcellulose, Carboxy-propylcellulose, und jeweils Salz davon (z.B. Na-Salze) sowie Guar-Galacto- mannan-Derivate und dergleichen. Im trockenen Zustand wird der Durchmesser des Bündels dadurch nur um wenige Millimeter erhöht, was eine leichte Einführung des Bündels in den Dialysator erlaubt. Je nachdem, wo das Polymer aufgebracht wurde, kann die Folie im Dialysator verbleiben oder wieder entfernt werden. Der Polymerring oder -streifen kann im Dialysator verbleiben. Wird der Dialysator bspw. vor der Behandlung gespült, saugt sich das Polymer mit dem Wasser der Spüllösung voll und sein Volumen vergrößert sich. Vernetzte Polyacrylsäure nimmt das 500- bis 1000-fache des Eigengewichts an Wasser auf. Das vergrößerte Volumen des Füllstoffringes wirkt dem Dialysierflüssigkeitsstrom entgegen und bewirkt auf der Anströmseite eine Druckerhöhung und auf der Abströmseite eine Druckverringerung. Die Volumenvergrö-βerung wird vorzugsweise so eingestellt, dass der Blutfluss in den Kapillaren nicht beeinflusst wird. Auf der Abströmseite wird so durch den entstandenen Unterdruck weit mehr Wasser durch die Kapillarwand aus dem Blut auf die Dialysierflüssigkeitsseite gedrückt, als bei einem herkömmlichen Dialysator. Dieses fehlende Wasser kann der Dialysierflüssigkeit durch eine Volumensteuerung des Dialysegeräts an der Anströmseite des Dialysators entzogen und vom Blut aufgenommen. Damit entsteht eine hohe interne Filtration im Dialysator, ohne dass ein zusätzlicher apparativer Aufwand bspw. zum Steuern und/oder Regeln einer Re-infusion von außen ergänzt werden muss.

Im Folgenden werden Aufbau und Herstellung eines Dialysators gemäß dem ersten Ausführungsbespiel anhand der Figuren 2 bis 4 näher erläutert.

Fig. 2 zeigt eine schematische Darstellung eines Faserbündels mit einem Füllstoffstreifen gemäß dem ersten Ausführungsbespiel. Auf ein Bündel aus einer Vielzahl von Kapillaren 10 (Faserbündel) wird direkt oder auf eine das Faserbündel umschließende Hüllfolie ein streifenförmiger Füllstoff 20 aus einem trockenen Polymer der vorgenannten Art aufgebracht, welches Superadsorbereigenschaften aufweist und damit nach Aktivierung ein deutlich vergrößertes Volumen einnimmt.

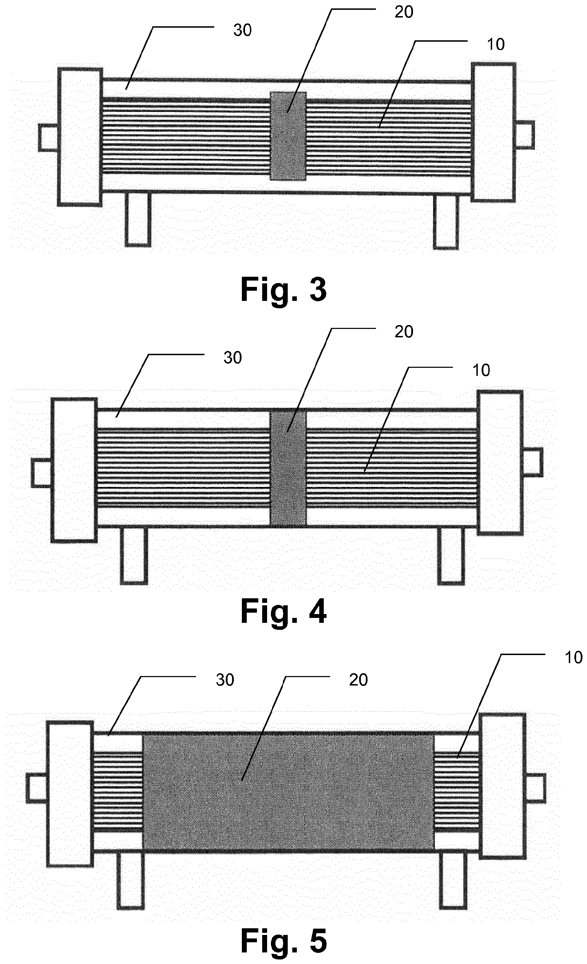

Fig. 3 zeigt eine schematische Darstellung eines Dialysators mit Dialysatorgehäuse 30 und eingeführtem Faserbündel aus den Kapillaren 10 und dem streifenförmigen Füllstoff 20 gemäß dem ersten Ausführungsbespiel. Das Faserbündel wird dabei in das Dialysatorgehäuse 30 eingeführt (bspw. gezogen). Die geringe Ausdehnung des trockenen Polymers ermöglicht ein erleichtertes Einführen.

Fig. 4 zeigt eine schematische Darstellung des Dialysators mit dem Dialysatorgehäuse 30 und dem eingeführten Faserbündel aus den Kapillaren 10 und dem streifenförmigen Füllstoff 20 nach Aktivierung der Volumenvergrößerung bspw. durch Inkontaktbringen mit Wasser. Kommt das Faserbündel mit Wasser in Berührung, so nimmt das Polymer des Füllstoffs 20 Wasser auf und quillt. Somit bildet sich eine Strömungsverengung für die Dialysierflüssigkeit aus, die dann zu dem in Fig. 1(c) gezeigten Druckprofil mit Druckgradientenumkehr und verbesserter Rückfiltration führt.

Fig. 5 zeigt eine schematische Darstellung eines Dialysators mit Dialysator gehäuse 30 und eingeführtem Faserbündel aus Kapillaren 10 mit einem in Längsrichtung des Dialysatorgehäuses 30 vergrößerten streifenförmigen Füllstoff 20 gemäß einem zweiten Ausführungsbespiel nach dessen Volumenvergrößerung. Die Breite des streifenförmigen Füllstoffs (Polymerstreifen) 20 kann sich somit auch über nahezu die gesamte Länge des Dialysatorgehäuses 30 erstrecken. Diese Maßnahme bewirkt eine Erhöhung der Packungsdichte des Dialysators, wodurch eine Verbesserung der Leistungsdaten des Dialysators im Gesamten erzielt werden kann.

In den vorgenannten Ausführungsbeispielen kann das Polymer des Füllstoffs 20 entweder in einer wasserdurchlässigen Folie verpackt oder als gelartige Paste aufgebracht werden. In der WO 2003020824 A1 ist bspw. eine hierfür geeignete selbstklebende Gelmatrix auf Polyacrylsäurebasis offenbart, die als Vernetzungsagenz Polyvinylpyrrolidon (PVP) enthält.

Ferner kann die Kinetik der Quellung bspw. über den Polymergehalt und/oder die Partikelgröße eingestellt werden.

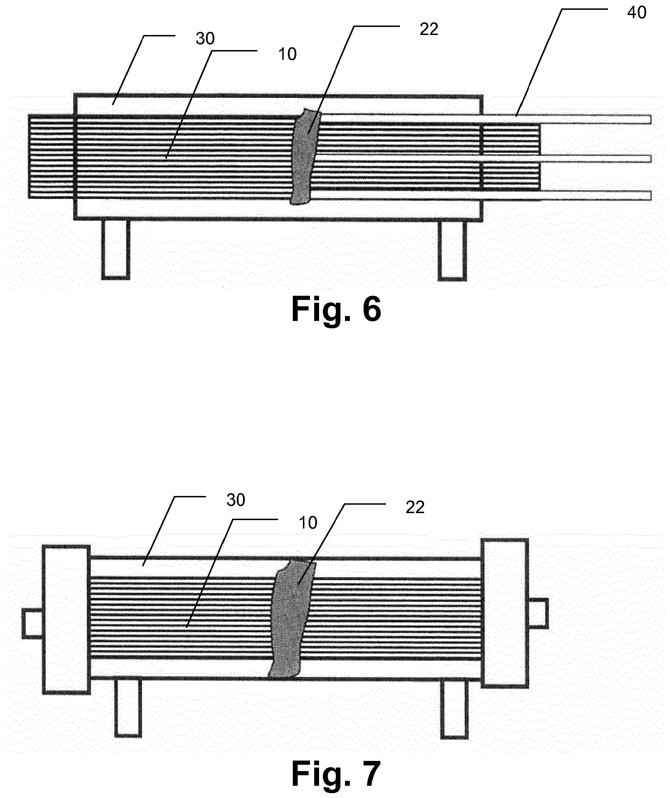

Nachfolgend wird ein alternatives drittes Ausführungsbeispiel mit einer anderen Ausgestaltung des Füllstoffs als Polymerschaum unter Bezugnahme auf die Figuren 6 und 7 beschrieben.

Fig. 6 zeigt eine schematische Darstellung eines Dialysators mit geöffnetem Dialysator Gehäuse 30 ohne Endkappen mit eingeführten Düsen 40 zum Einbringen eines schaumartigen Füllstoffs 22 gemäß dem dritten Ausführungsbespiel.

Das Polymer des schaumartigen Füllstoffs 22 wird über die langen Düsen 40 in den gewünschten Bereich des Dialysatorgehäuses 30 eingebracht bzw. eingespritzt. Durch die chemische Reaktion bildet sich während der Härtung ein Gas, das das Polymer in eine Schaumform bringt und so eine Volumenvergrößerung bewirkt. Das Kunststoffschaumsystem kann dabei bspw. eines der üblichen in der Medizintechnik eingesetzten Schaumsysteme sein, mit bspw. einem Zwei-Komponenten-Polyurethanschaum, einem Zwei-Komponenten-Polyurethan-Aerosoldosenschaum und/oder einem Zwei-Komponenten-Epoxidharzschaum. Alternativ können auch Silikonschaumsysteme eingesetzt werden oder ein quellbares Polymer gemäß den ersten beiden Ausführungsbeispielen kann in einen Schaum eingebracht werden.

Fig. 7 zeigt eine schematische Darstellung des Dialysatorgehäuses 30 mit dem eingebrachtem schaumartigen Füllstoff 22 gemäß dem dritten Ausführungsbespiel nach der Volumenvergrößerung.

Zusammenfassend wurden ein Dialysator und Verfahren zur Herstellung eines solchen beschrieben, wobei der Dialysator ein röhrenförmiges Dialysatorgehäuse aufweist, in dessen Innenraum eine Vielzahl von sich jeweils in Längsrichtung des Dialysatorgehäuses 30 erstreckenden, quer zur Längsrichtung nebeneinander angeordneten Kapillaren 10 angeordnet sind, wobei zwischen der Innenwand des Dialysatorgehäuses 30 und den Kapillaren 10 ein Füllstoff 20, 22 mit volumenvergrößernder Eigenschaft angeordnet ist.

## Patentansprüche

1. Dialysator mit einem röhrenförmigen Dialysatorgehäuse (30), in dessen Innenraum eine Vielzahl von sich jeweils in Längsrichtung des Dialysatorgehäuses (30) erstreckenden, quer zur Längsrichtung nebeneinander angeordneten Kapillaren (10) angeordnet sind, wobei zwischen der Innenwand des Dialysatorgehäuses (30) und den Kapillaren (10) ein Füllstoff (20, 22) mit volumenvergrößernder Eigenschaft angeordnet ist, welcher nach dem Einbringen in das Dialysatorgehäuse (30) expandiert, **dadurch gekennzeichnet, dass**
die Kapillaren (10) mit einer Folie umwickelt sind und der Füllstoff (20, 22) zwischen der Folie und der Innenwand des Dialysatorgehäuses (30) angeordnet ist, wobei
der Füllstoff (20) von einer wasserdurchlässigen Folie umgeben ist oder als gelartige Paste ausgebildet ist oder als in das Dialysatorgehäuse (30) einspritzbarer Polymerschaum ausgebildet ist.

2. Dialysator nach Anspruch 1, wobei der Füllstoff (20, 22) so ausgestaltet und dessen Volumenvergrößerung so eingestellt ist, dass diese beim Betrieb des Dialysators die Durchflussmenge der Kapillare (10) nicht beeinflusst.

3. Dialysator nach einem der Ansprüche 1 oder 2, wobei der Füllstoff (20) ein wasserquellbarer Füllstoff ist.

4. Dialysator nach Anspruch 3, wobei der Füllstoff (20) ein wasserquellbares Polymer ist.

5. Dialysator nach Anspruch 4, wobei der Füllstoff (20) ein Homo- oder Copolymer auf Grundlage von zumindest einem aus Acrylsäure, Methacrylsäure, Acrylamid, Methacrylamid, Acrylat und Methacrylat ist.

6. Dialysator nach einem der vorgenannten Ansprüche, wobei der Füllstoff (20) streifenförmig ausgebildet ist und quer zu der Längsrichtung um die Vielzahl von Kapillaren (10) gewickelt ist.

7. Dialysator nach Anspruch 6, wobei sich der streifenförmige Füllstoff (20) in der Längsrichtung über die gesamte Länge des Dialysatorgehäuses (30) erstreckt.

8. Dialysator nach Anspruch 7, wobei der in das Dialysatorgehäuse (30) einspritzbare Polymerschaum ein Zwei-Komponenten-Polymerschaum ist.

9. Verfahren zur Herstellung eines Dialysators, insbesondere zur Herstellung des Dialysators nach Ansprüchen 1 bis 8, mit den Schritten:
- Aufbringen eines von einer wasserdurchlässigen Folie umgebenen oder als gelartige Paste ausgebildeten Füllstoffs (20) mit volumenvergrößernder Eigenschaft auf ein Bündel von Kapillaren (10), das mit einer Folie umwickelt ist,
- Einbringen des Bündels mit dem auf der Folie aufgebrachten Füllstoff (20) in ein Dialysatorgehäuse (30), sodass der Füllstoff (20, 22) zwischen der Folie und der Innenwand des Dialysatorgehäuses (30) angeordnet ist, und
- nachfolgendes Aktivieren eines volumenvergrößernden Mechanismus des Füllstoffs, so dass der Füllstoff expandiert.

10. Verfahren nach Anspruch 9, wobei der volumenvergrößernde Mechanismus durch Zuführen einer Flüssigkeit aktiviert wird.

## Claims

1. A dialyzer comprising a tubular dialyzer housing (30) in the interior of which a plurality of capillaries (10) each extending in the longitudinal direction of the dialyzer housing (30) and being juxtaposed transversely to the longitudinal direction is arranged, with a filler (20, 22) having a volume-increasing property being arranged between the inner wall of the dialyzer housing (30) and the capillaries (10) and which expands after introduction into the dialyzer housing (30), **characterized in that**
the capillaries (10) are wrapped with a film and the filler (20, 22) is arranged between the film and the inner wall of the dialyzer housing (30), wherein
the filler (20) is surrounded by a water-permeable film or the filler (20) is a gel-type paste or a polymer foam adapted to be injected into the dialyzer housing (30).

2. The dialyzer according to claim 1, wherein the filler (20, 22) is configured such and the increase in volume thereof is set such that this does not influence the flow rate of the capillaries (10) when the dialyzer is operated.

3. The dialyzer according to one of the claims 1 or 2, wherein the filler (20) is a filler capable of swelling by water.

4. The dialyzer according to claim 3, wherein the filler (20) is a polymer capable of swelling by water.

5. The dialyzer according to claim 4, wherein the filler (20) is a homopolymer or copolymer on the basis of at least one out of acrylic acid, methacrylic acid, acrylamide, methacrylamide, acrylate and methacrylate.

6. The dialyzer according to one of the preceding claims, wherein the filler (20) is strip-shaped and is wound around the plurality of capillaries (10) transversely to the longitudinal direction.

7. The dialyzer according to claim 6, wherein the strip-shaped filler (20) extends in the longitudinal direction over the total length of the dialyzer housing (30).

8. The dialyzer according to claim 7, wherein the polymer foam injectable into the dialyzer housing (30) is a two-component polymer foam.

9. A method of manufacturing a dialyzer, in particular of manufacturing the dialyzer according to claims 1 to 8, comprising the steps of:
- applying a filler (20), which is surrounded by a water-permeable film or which is a gel-type paste and has a volume-increasing characteristic to a bundle of capillaries (10) around which a film is wrapped,
- introducing the bundle including the filler (20) applied to the film to a dialyzer housing (30), such that the filler (20, 22) is arranged between the film and the inner wall of the dialyzer housing (30), and
- subsequently activating a volume-increasing mechanism of the filler so that the filler expands.

10. The method according to claim 9, wherein the volume-increasing mechanism is activated by supplying a liquid.

## Revendications

1. Dialyseur avec un boîtier de dialyseur (30) tubulaire, dans l'espace intérieur duquel est agencée une pluralité de capillaires (10) s'étendant respectivement dans le sens longitudinal du boîtier de dialyseur (30), agencés transversalement au sens longitudinal les uns à côté des autres, dans lequel une substance de remplissage (20, 22) avec une propriété augmentant le volume est agencée entre la paroi intérieure du boîtier de dialyseur (30) et les capillaires (10), laquelle se dilate après l'introduction dans le boîtier de dialyseur (30), **caractérisé en ce que**
les capillaires (10) sont enroulés avec un film et la substance de remplissage (20, 22) est agencée entre le film et la paroi intérieure du boîtier de dialyseur (30), dans lequel
la substance de remplissage (20) est entourée par un film perméable à l'eau ou est réalisée comme pâte en gel ou est réalisée comme mousse de polymère pouvant être injectée dans le boîtier de dialyseur (30).

2. Dialyseur selon la revendication 1, dans lequel la substance de remplissage (20, 22) est configurée et son augmentation de volume est réglée de sorte que celle-ci n'influence pas le débit des capillaires (10) lors du fonctionnement du dialyseur.

3. Dialyseur selon l'une quelconque des revendications 1 ou 2, dans lequel la substance de remplissage (20) est une substance de remplissage hydrogonflable.

4. Dialyseur selon la revendication 3, dans lequel la substance de remplissage (20) est un polymère hydrogonflable.

5. Dialyseur selon la revendication 4, dans lequel la substance de remplissage (20) est un homo- ou copolymère sur la base d'au moins un parmi l'acide acrylique, acide méthacrylique, acrylamide, méthacrylamide, acrylate et méthacrylate.

6. Dialyseur selon l'une quelconque des revendications précédentes, dans lequel la substance de remplissage (20) est réalisée en forme de bande et est enroulée transversalement au sens longitudinal autour de la pluralité de capillaires (10).

7. Dialyseur selon la revendication 6, dans lequel la substance de remplissage (20) en forme de bande s'étend dans le sens longitudinal sur la longueur entière du boîtier de dialyseur (30).

8. Dialyseur selon la revendication 7, dans lequel la mousse de polymère pouvant être injectée dans le boîtier de dialyseur (30) est une mousse de polymère à deux composants.

9. Procédé de fabrication d'un dialyseur, en particulier de fabrication du dialyseur selon les revendications 1 à 8, avec les étapes consistant à :
- appliquer une substance de remplissage (20) entourée par un film perméable à l'eau ou réalisée comme pâte en gel avec une propriété agrandissant le volume sur un faisceau de capillaires (10) qui est enroulé avec un film,
- introduire le faisceau avec la substance de remplissage (20) appliquée sur le film dans un boîtier de dialyseur (30) de sorte que la substance de remplissage (20, 22) soit agencée entre le film et la paroi intérieure du boîtier de dialyseur (30), et
- activer ensuite un mécanisme augmentant le volume de la substance de remplissage de sorte que la substance de remplissage se dilate.

10. Procédé selon la revendication 9, dans lequel le mécanisme augmentant le volume est activé par amenée d'un liquide.
